# FASCICULE DE BREVET EUROPEEN

(11) **EP 1 603 454 B1**
(45) Date de publication et mention de la délivrance du brevet: **05.08.2009**
(21) Numéro de dépôt: 04717656.5
(22) Date de dépôt: 05.03.2004
(51) Int. Cl.: A61B 3/12, A61B 5/00

(54) **PROCEDE ET DISPOSITIF DE CARTOGRAPHIE DU PH INTRA-RETINIEN, DISPOSITIF DE PHOTOCOAGULATION DES ZONES DE LA RETINE PERIPHERIQUES.**
VERFAHREN UND GERÄT ZUR MEHRDIMENSIONALEN DARSTELLUNG DES RETINALEN PH'S UND GERÄT ZUR PHOTOCOAGULATION DER RETINA
METHOD AND DEVICE FOR THE MAPPING OF INTRARETINAL PH AND DEVICE FOR THE PHOTOCOAGULATION OF PERIPHERAL RETINAL AREAS

(30) Priorité: 14.03.2003 FR 0303174
(43) Date de publication de la demande: 14.12.2005
(73) Titulaire: INSTITUT NATIONAL DE LA SANTE ET DE LA RECHERCHE MEDICALE (INSERM), 75654 Paris Cédex 13 (FR); CENTRE HOSPITALIER REGIONAL ET UNIVERSITAIRE DE LILLE, 59037 Lille Cédex (FR)
(72) Inventeur: MORDON, Serge, F-59491 Villeneuve d'Ascq (FR); ROCHON, Philippe, F-59450 Sin Le Noble (FR)
(74) Mandataire: Loisel, Bertrand
(86) Numéro de dépôt international: PCT/FR2004/000534
(87) Numéro de publication internationale: WO 2004/082473

(56) Documents cités:
- WO-A-00/47107
- WO-A-92/12412
- WO-A-02/069789
- US-A- 5 093 266
- US-A- 5 557 349
- US-A- 5 975 697
- US-B1- 6 371 615
- SCHWEITZER D ET AL: "IMAGING SPECTROMETRY IN OPHTHALMOLOGY-PRINCIPLE AND APPLICATIONS INMICROCIRCULATION AND INVESTIGATION OF PIGMENTS" OPHTHALMIC RESEARCH, BASEL, CH, vol. 28, no. SUPPL 2, 1996, pages 37-44, XP001037363

## Description

La présente invention est relative au domaine de la cartographie intra-rétinienne.

Les cartographies intra-rétiniennes sont habituellement réalisées par angiographie ou en réalisant des photographies du fond d'oeil. Cependant, ces techniques fournissent des résultats qualitatifs et quelquefois difficiles à interpréter.

Le document WO92/12412 décrit par ailleurs un dispositif et un procédé de mesure du pH d'une cible, pouvant être utilisés par voie endoscopique et utilisant des marqueurs fluorescents. Le procédé décrit dans ce document s'affranchit des conditions de mesure en travaillant sur des rapports entre fluorescences (et non sur fluorescences elles-mêmes). Cependant, elle ne permet pas d'obtenir une cartographie du pH, en deux dimensions, d'une zone cible.

La présente invention vise à fournir un procédé et un dispositif non invasifs de cartographie par fluorescence du pH intra-rétinien ne mettant en oeuvre que des techniques et moyens bien maîtrisés, et permettant d'obtenir une représentation fidèle et directement utilisable pour un diagnostic ultérieur.

Suivant un premier aspect, l'invention propose un procédé de cartographie du pH intra-rétinien, suivant lequel, après marquage de la rétine par un marqueur fluorescent dont le spectre d'émission de fluorescence présente un pic dont l'amplitude dépend du pH :
a)on éclaire la rétine avec un premier rayonnement d'excitation à spectre étroit centré sur une longueur d'onde appartenant au spectre d'excitation du marqueur fluorescent, et on forme une première image de la fluorescence émise par la rétine, sous forme d'une matrice bidirectionnelle de pixels, à la longueur d'onde du pic du spectre d'émission de fluorescence ;
b) on éclaire la rétine avec un second rayonnement d'excitation à spectre étroit centré sur une longueur d'onde du spectre d'excitation du marqueur fluorescent différente de celle du premier rayonnement, et on forme une seconde image de la fluorescence émise par la rétine, sous forme de la matrice bidimensionnelle de pixels, à la longueur d'onde du pic du spectre d'émission de fluorescence ;
c) on calcule le rapport, en chaque pixel, entre les niveaux de fluorescence émise en réponse aux deux longueurs d'onde d'excitation respectives, ce rapport étant représentatif du pH de la rétine, et on en déduit une carte du pH de la rétine.

Suivant un second aspect, l'invention propose un procédé de cartographie du pH intra-rétinien, après marquage de la rétine par un marqueur fluorescent dont le spectre d'émission de fluorescence présente un pic dont l'amplitude dépend du pH . Ce procédé ne fait appel qu'à un seul rayonnement d'excitation et comporte les étapes suivantes :
a) on éclaire la rétine avec un rayonnement d'excitation à spectre étroit centré sur une longueur d'onde appartenant au spectre d'excitation du marqueur fluorescent,
b) on forme une première image de la fluorescence émise par la rétine, sous forme d'une matrice bidirectionnelle de pixels, à la longueur d'onde du pic du spectre d'émission de fluorescence ;
c) on forme une seconde image de la fluorescence émise par la rétine, sous forme d'une matrice bidirectionnelle de pixels, à une longueur d'onde correspondant à un point isosbestique du spectre d'émission de fluorescence ;
d) on calcule le rapport, en chaque pixel, entre les niveaux de fluorescence émise aux deux longueurs d'onde respectives appartenant au spectre d'émission, ce rapport étant représentatif du pH de la rétine, et on en déduit une carte du pH de la rétine.

Suivant un troisième aspect, l'invention porte sur un dispositif de cartographie du pH intra-rétinien d'une rétine marquée par un marqueur fluorescent dont le spectre d'émission de fluorescence présente un pic d'intensité dont la hauteur dépend du pH.

On sait que l'ischémie rétinienne, qui se caractérise entre autre, par une acidification de la neurorétine interne, peut conduire à la formation de néo vaisseaux rétiniens. Ces vaisseaux rétiniens peuvent saigner, se fibroser, et entraîner des problèmes de baisse importante d'acuité visuelle. L'utilisation d'un procédé et d'un dispositif suivant l'invention permet une meilleure quantification de l'ischémie que les méthodes et procédés de l'art antérieur, et facilite le diagnostic par un praticien et la détermination d'indications pour un traitement ultérieur.

Suivant un quatrième aspect, l'invention porte sur un dispositif de photo coagulation des zones de rétine périphériques, pouvant incorporer les éléments du dispositif de cartographie. Ce dispositif comprend les mêmes éléments que le dispositif de cartographie du pH intra-rétinien et un laser muni de moyens de focalisation et d'orientation d'un faisceau de sortie vers le ou les points de la rétine représentés, sur l'image de cartographie du pH fournie par le dispositif, par des pixels correspondant à des pH inférieurs à un seuil donné.

D'autres caractéristiques et avantages de l'invention apparaîtront au cours de la description suivante de formes de réalisation, données à titre d'exemple non limitatif, et en référence aux dessins joints.

Sur les dessins :
- la figure 1 est une vue schématique d'un mode de réalisation d'un dispositif de cartographie du pH intra-rétinien dans un mode de mise en oeuvre de l'invention ;
- la figure 2 représente le spectre de l'excitation de fluorescence de la BCECF en fonction du pH ;
- la figure 3 représente le spectre de la fluorescence d'émission de la BCECF, en fonction du pH ;
- la figure 4 représente le spectre de la fluorescence d'émission de la SNAFL-1 en fonction du pH ;
- la figure 5 est une courbe de correspondance entre les rapports de fluorescence et le pH ;
- la figure 6 est une vue schématique d'un dispositif de coagulation des zones de rétine périphériques selon l'invention ;
- la figure 7 est une vue schématique d'un autre dispositif de photo coagulation selon l'invention.

Sur les différentes figures, les mêmes références désignent des éléments identiques ou similaires.

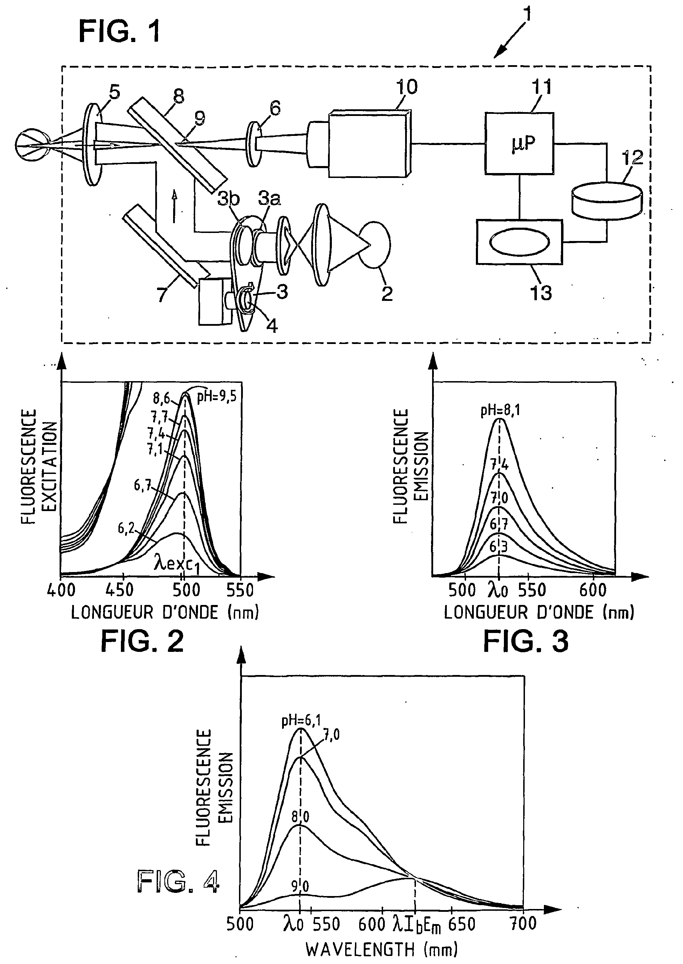

La figure 1 représente une vue schématique d'un dispositif 1 de cartographie du pH intra-rétinien d'une rétine marquée par un marqueur fluorescent dans un mode de réalisation de l'invention, comprenant une source lumineuse 2 dont le spectre du rayonnement contient une partie au moins du spectre d'excitation du marqueur fluorescent utilisé. Cette source lumineuse 2 est associée à un système de filtres 3. Ce système de filtres comporte deux filtres 3a et 3b, que l'on peut sélectionner successivement. Ces filtres 3a et 3b sont à bandes étroites et centrés chacun sur une longueur d'onde distincte, respectivement λexc1 et λexc2, du spectre d'excitation du marqueur fluorescent. On passe d'un filtre à l'autre en actionnant un bras 4 portant les 2 filtres.

La source 2 est par exemple une lampe à Xénon. La puissance est par exemple 150W. La bande des filtres 3a et 3b nm est par exemple de 10nm de largeur à mi-hauteur. On pourrait également utiliser, à la place de la source 2 associée au système de filtres 3, des diodes laser respectivement de longueur d'onde λexc1 et λexc2.

Le dispositif 1 comporte en outre une optique de focalisation 5 permettant de focaliser le rayonnement sur la rétine humaine.

Sur le trajet de retour de la lumière, le dispositif comprend un filtre 6 adapté pour ne laisser passer qu'une bande étroite contenant la longueur d'onde λ0 du pic du spectre d'émission de fluorescence qui correspond au pic de sensibilité du pH. Ce filtre reçoit ainsi la fluorescence de la rétine éclairée par la source lumineuse.

Dans un mode de mise en oeuvre avantageux, le dispositif est incorporé à un angio-rétinographe et comprend en outre 2 miroirs de repliement du trajet de retour 7,8. Une ouverture 9 du miroir 8 permet le passage de la fluorescence d'émission, permettant de séparer la voie incidente de la voie de retour de la rétine.

Le dispositif 1 comporte en outre une caméra matricielle 10. de formation d'une image 2D. Cette caméra fournit une image avec en chaque pixel, une valeur représentative du niveau de fluorescence.

Le dispositif comprend également des moyens de calcul 11 du rapport entre les valeurs en chaque pixel pour deux images fournies. Il est également muni de moyens de mémorisation 12 de ces valeurs. Le dispositif 1 comprend également en général des moyens de visualisation 13 d'une image.

La caméra peut être par exemple une caméra CCD, sensible pour des longueurs d'onde du spectre d'émission de la BCECF. Elle peut comprendre 512x512 pixels. Les moyens de calcul 11, de mémorisation 12 et de visualisation 13 sont par exemple le microprocesseur, le disque et l'écran d'un ordinateur.

On procède comme suit dans un mode de mise en oeuvre du premier procédé de cartographie selon l'invention. On injecte préalablement dans une veine un marqueur fluorescent dont la fluorescence dépend du pH. De nombreux marqueurs fluorescents dépendant du pH sont utilisables (SNAFL, NSARF, HPTS, fluorescéines et carbofluorescéines...). Le mode de mise en oeuvre présenté ci-dessous utilise la BCECF(2',7'-bis(2-carboxyethyl)-5(et6)carboxyfluorescéine), dont la gamme de sensibilité se situe dans les pH physiologiques. La BCPCF (2',7'-bis(2-carboxypropyl)-5(et6) carboxyfluorescéine) peut également être utilisée. La BCPCF est un dérivé de la BCECF, dans lequel le groupement carboxyéthyle a été remplacé par le groupement carboxypropyle. La BCPCF présente une longueur d'onde du point isosbestique du spectre d'excitation de 454 nm, et une longueur d'onde du point isosbestique du spectre d'émission de 504 nm.

La quantité de BCECF injectée se situe par exemple dans la plage allant de 0,1 à 10mg/kg du corps du patient. Dans le mode de mise en oeuvre présenté ci-dessous, elle est de 1mg/kg.

Une fois écoulé un délai après l'injection suffisant pour que la rétine ait reçu le marqueur, on éclaire la rétine avec un premier rayonnement d'excitation à spectre étroit (10 nm de largeur à mi-hauteur) centré sur une longueur d'onde λexc1 du spectre d'excitation du marqueur fluorescent. Ce rayonnement correspond au rayonnement de la source lumineuse 2 filtrée par le premier filtre de longueur d'onde λexc1 du système de filtres 3. On forme simultanément, sur l'organe sensible de la caméra 10, une première image de la fluorescence émise par la rétine. Cette première image, sous forme d'une matrice bidirectionnelle de pixels, représente la fluorescence de la rétine filtrée par le système de filtres 6 à la longueur d'onde λ0 On stocke cette image sous forme numérique dans les moyens de mémorisation 12.

La figure 2 représente le spectre de fluorescence d'excitation de la BCECF, en fonction du pH. Il représente les variations de l'effet de fluorescence aux différentes longueurs d'onde d'excitation.

La figure 3 représente le spectre d'émission de la BCECF, en fonction du pH, présentant un pic à une longueur d'onde λ0

Avantageusement, λexc1 est égale à la longueur d'onde du pic du spectre d'excitation du marqueur fluorescent. Dans le cas de la BCECF. dont le spectre d'excitation est représenté en figure 2, on pourra donc prendre λexc1 égale à 490 nm.

Puis on remplace le premier filtre par le second filtre, centré sur λexc2, en déplaçant le bras 4. On éclaire la rétine avec un second rayonnement d'excitation à spectre étroit centré sur la longueur d'onde λexc2 du spectre d'excitation du marqueur fluorescent différente de celle du premier rayonnement. On forme une seconde image de la fluorescence émise par la rétine, sous forme d'une matrice bidimensionnelle de pixels, à nouveau à la longueur d'onde λ0 .

On pourra prendre par exemple Àexc2 égale à 470 nm.

Avantageusement dans une autre mise en oeuvre de l'invention, on pourra également choisir Àexc2 égale à la longueur d'onde de 450 nm du point isobestique du spectre d'excitation (point présentant un même taux d'absorption quelque soit le pH) représenté en figure 2.

On calcule à l'aide du microprocesseur 11, le rapport, en chaque pixel, entre les niveaux de fluorescence à la longueur d'onde λ0 après mémorisation éventuelle, émise en réponse aux deux longueurs d'onde d'excitation respectives λexc1 et λexc2 ;

Enfin, on détermine une carte du pH de la rétine à partir de ce rapport, qui est représentatif du pH de la rétine. On peut visualiser cette image sur l'écran 13.

La mesure du pH est fiable et non dépendante des conditions de mesure, car elle utilise un rapport d'intensité de fluorescence pour deux longueurs d'onde d'excitation différentes. On obtient la valeur du pH à partir du rapport de la fluorescence calculé, en utilisant une courbe de correspondance rapport/pH prédéterminée.

Dans un autre mode de mise en oeuvre de l'invention, on utilise un dispositif suivant une variante dont le système 3 de filtres comporte au moins un filtre à bande étroite (largeur à mi-hauteur de 10 nm) centré sur une longueur d'onde λexc du spectre d'excitation du marqueur fluorescent. Le dispositif comporte un système de filtres, à la place du filtre 6, adapté pour filtrer successivement, à deux longueurs d'onde différentes respectives λ0 et λIbEm la fluorescence de la rétine éclairée par la source lumineuse. λ0 et λIbEm correspondent respectivement à la longueur d'onde du pic, et à celle du point isosbestique, du spectre d'émission de fluorescence. La figure 4 présente le spectre d'émission du marqueur fluorescent SNAFL-1 pour lequel λ0=540 nm et λIbEm=635 nm. Le même système que précédemment pour basculer d'un filtre à l'autre peut être utilisé.

Le procédé mis en oeuvre à l'aide de ce dernier dispositif est le suivant.

On injecte un marqueur fluorescent dépendant du pH. Puis on éclaire la rétine avec un rayonnement d'excitation à spectre étroit (typiquement largeur à mi-hauteur de 10 nm) centré sur la longueur d'onde λexc du spectre d'excitation du marqueur fluorescent, à l'aide de la source lumineuse 2 dont l'émission est filtrée par le filtre 3 centré sur la longueur d'onde Àexc. La caméra 10 forme une première image de la fluorescence émise par la rétine, sous forme d'une matrice bidirectionnelle de pixels, à la longueur d'onde du pic du spectre d'émission de fluorescence λ0 On stocke les données correspondantes à l'aide des moyens de mémorisation 12.

Puis on remplace le premier filtre du système de filtres par le second filtre centré sur λIbEm et on forme une seconde image de la fluorescence émise par la rétine, sous forme d'une seconde matrice bidirectionnelle de pixels, à la longueur d'onde λIbEm correspondant au point isosbestique du spectre d'émission.

Avantageusement, on peut utiliser un système optique permettant à la caméra CCD de former les deux images de la fluorescence.

Enfin on détermine une carte du pH de la rétine à partir du rapport calculé, en chaque pixel, entre les fluorescences émises aux deux longueurs d'onde du spectre d'émission de fluorescence, ce rapport étant représentatif du pH de la rétine.

Dans un mode de mise en oeuvre avantageux de l'invention, on peut choisir comme longueur d'onde d'excitation Àexc la longueur d'onde correspondant au pic du spectre d'excitation.

Par exemple, si on utilise le marqueur fluorescent C-SNAFL-1, on peut mettre en oeuvre le procédé ci-dessus décrit en utilisant une excitation unique à 514 nm et en formant deux images correspondant à la fluorescence aux longueurs d'ondes respectives de 540 nm et 635 nm.

Les mesures effectuées au cours d'un procédé selon l'invention ont lieu dans un délai après l'injection compris entre 3 minutes minimum et 3 heures maximum, habituellement de quelques minutes.

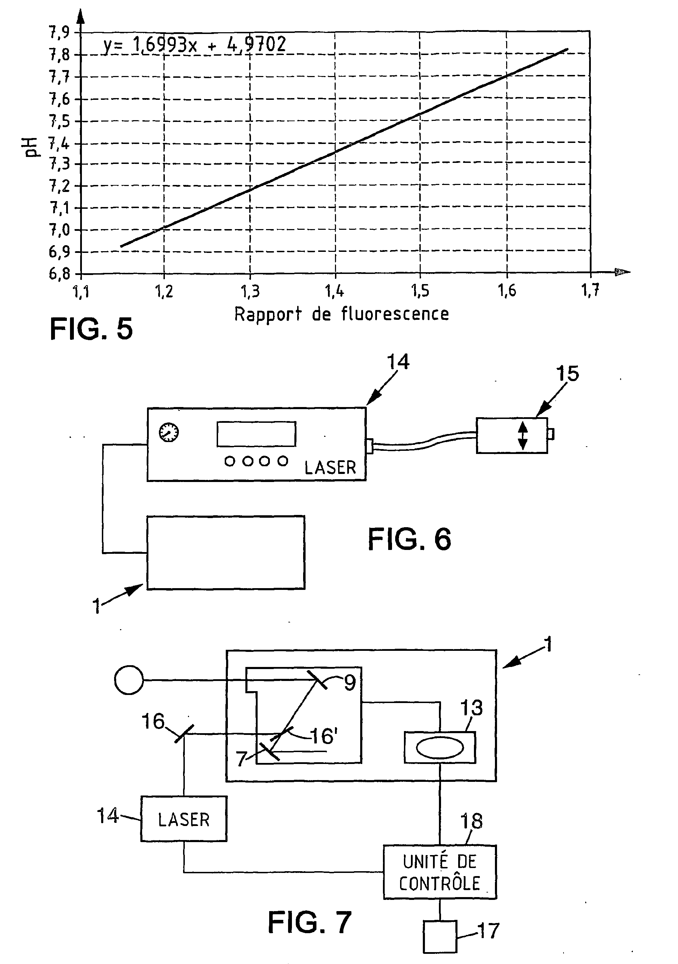

Une table de correspondance entre des rapports de fluorescence et le pH peut par exemple être établie au moyen d'une mesure préalable en reproduisant les conditions d'emploi prévues par comparaison avec les données fournies par une électrode de pH, sur un tissu semblable et dans des conditions d'éclairage identiques à celles des mesures qui seront effectuées selon le procédé. La figure 6 donne un exemple de courbe de correspondance établie suite à une telle mesure préalable. Il existe par ailleurs d'autres principes connus pour établir cette correspondance, par exemple celui présenté dans l'article "Fluorescence Behavior of Single-Molecule pH Sensors", de S. Brasselet et W.E. Moerner, Research Paper, Single Molecules (2000).

Le dispositif peut être complété par des moyens de photo coagulation de zones de la rétine en fonction de la cartographie du pH intra-rétinien selon l'invention, en ajoutant un laser de traitement au dispositif de cartographie.

La photo coagulation de zones de la rétine notamment périphériques, permet de détruite les zones ischémiques, qui entraînent un appel de néo vaisseaux.

On peut procéder de la façon suivante, à l'aide du dispositif représenté en figure 6 : après avoir mis dans l'oeil une goutte de collyre anesthésique, on réalise la cartographie du pH de l'oeil selon l'un des procédés de l'invention, à l'aide du dispositif 1 représenté en figure 1.

Puis on repère les pixels correspondant aux points de la rétine dont le pH est inférieur à un seuil donné et on envoie une impulsion de laser sur ces seuls points.

Le seuil de pH considéré est par exemple égal à 7,2. On traite au laser ces points de la rétine, correspondant aux zones ischémiques.

Pour cela, dans le cas représenté en figure 6, on utilise, couplé au dispositif de cartographie du pH intra-rétinien 1, un laser 14 dont on peut régler la puissance de sortie et la largeur d'impulsions, et muni de moyens de focalisation et d'orientation 15. Le laser peut par exemple être dirigé à l'aide d'une manette sur les points de la rétine identifiés, à travers une lampe à fente ou un biomicroscope connecté(e) au laser.

Le laser peut être également directement couplé au rétinographe utilisé dans le mode de réalisation du dispositif 1 décrit plus haut.

La figure 7 représente un dispositif de photocoagulation dans un mode de réalisation de l'invention. Le laser est dans ce cas directement couplé, par l'intermédiaire de miroirs de repliement supplémentaires 16 et 16', au rétinographe, faisant partie du dispositif de cartographie 1 décrit ci-dessus dans un mode de mise en oeuvre de l'invention. Sur la figure 8 représentée, seuls l'écran de visualisation 13 et les miroirs 7 et 9 du dispositif 1 ont été représentés. Une manette ou un mini-manche 17 relié à une unité de contrôle 18, elle-même connectée à l'écran de visualisation 13, par exemple l'écran du rétinographe, permet de déplacer le faisceau laser sur la zone dont le pH est visualisé en temps réel sur l'écran 13.

Le laser peut être par exemple un laser argon monochromatique vert, ou bien un laser Nd:YAG doublé à 532 nm. Dans les cas d'opacité de la rétine, on peut utiliser un laser krypton. Les impacts sur les points identifiés sur la cartographie, sont définis par les moyens de focalisation entre 200 µm et 500 µm de diamètre, avec un temps d'exposition généralement de 0,1 à 0,2 s.

La puissance du laser utilisé est par exemple de 100 à 500 mW, et est ajustée en fonction de l'effet et également en fonction d'une éventuelle cataracte atténuant le faisceau laser. On peut réaliser quatre à six séances de 500 impacts chacune en cherchant à obtenir un aspect rétinien d'un blanc franc.

## Revendications

1. Procédé de cartographie du pH intra-rétinien d'une rétine marquée par un marqueur fluorescent dont le spectre d'émission de fluorescence à une amplitude qui dépend du pH, **caractérisé en ce que** :
a)on éclaire la rétine avec un premier rayonnement d'excitation à spectre étroit centré sur une longueur d'onde appartenant au spectre d'excitation du marqueur fluorescent, et on forme une première image de la fluorescence émise par la rétine, sous forme d'une matrice bidirectionnelle de pixels, à la longueur d'onde du pic du spectre d'émission de fluorescence ;
b) on éclaire la rétine avec un second rayonnement d'excitation à spectre étroit centré sur une longueur d'onde appartenant au spectre d'excitation du marqueur fluorescent différente de celle du premier rayonnement, et on forme une seconde image de la fluorescence émise par la rétine, sous forme de la matrice bidimensionnelle de pixels, à la longueur d'onde du pic du spectre d'émission de fluorescence ;
c) on calcule le rapport, en chaque pixel, entre les niveaux de fluorescence émise en réponse aux deux longueurs d'onde d'excitation respectives, ce rapport étant représentatif du pH de la rétine, et on en déduit une carte du pH de la rétine.

2. Procédé selon la revendication 1, dans lequel le premier rayonnement d'excitation est centré sur la longueur d'onde correspondant au pic du spectre d'absorption du marqueur fluorescent.

3. Procédé selon la revendication 1 ou la revendication 2, dans lequel le second rayonnement d'excitation est centré sur la longueur d'onde correspondant au point isosbestique du spectre d'excitation du marqueur fluorescent.

4. Procédé de cartographie du pH intra-rétinien d'une rétine marquée par un marqueur fluorescent dont le spectre d'émission de fluorescence dépend du pH, **caractérisé en ce que** :
a)on éclaire la rétine avec un rayonnement d'excitation à spectre étroit centré sur une longueur d'onde appartenant au spectre d'excitation du marqueur fluorescent,
b) on forme une première image de la fluorescence émise par la rétine, sous forme d'une matrice bidirectionnelle de pixels, à la longueur d'onde du pic du spectre d'émission de fluorescence ;
c) on forme une seconde image de la fluorescence émise par la rétine, sous forme d'une matrice bidirectionnelle de pixels, à une longueur d'onde correspondant à un point isobestique du spectre d'émission de fluorescence ;
d) on calcule le rapport, en chaque pixel, entre les niveaux de fluorescence émise aux deux longueurs d'onde respectives appartenant au spectre d'émission, ce rapport étant représentatif du pH de la rétine, et on en déduit une carte du pH de la rétine.

5. Procédé selon la revendication 4, dans lequel le rayonnement d'excitation est centré sur la longueur d'onde correspondant au pic du spectre d'absorption du marqueur fluorescent.

6. Dispositif de cartographie du pH intra-rétinien (1) d'une rétine marquée par un marqueur fluorescent dont le spectre d'émission de fluorescence présente un pic d'intensité dont la hauteur dépend du pH, **caractérisé en ce qu'**il comprend :
- une source lumineuse (2) munie de moyens (3, 3a, 3b) permettant la sélection d'une unique ou de deux successives distinctes longueurs d'onde du spectre d'excitation du marqueur fluorescent,
- une optique de focalisation (5) permettant de focaliser le rayonnement sur la rétine humaine,
- un ou des filtre(s) adapté(s) (6) pour filtrer à une ou des longueur(s) d'onde donnée(s) le spectre d'émission de fluorescence de la rétine éclairée par la source lumineuse,
- une caméra matricielle de formation d'une image 2D (10) fournissant une image avec en chaque pixel, une valeur représentative du niveau de fluorescence
- des moyens de mémorisation de ces valeurs (12),
- des moyens de calcul du rapport entre les valeurs en chaque pixel pour deux images fournies (11),
- des moyens de visualisation d'une image (13) dont chaque pixel a une valeur qui représente le rapport calculé, ce rapport étant représentatif du pH du point de la rétine représenté par le pixel.

7. Dispositif de cartographie du pH intra-rétinien (1) selon la revendication 6, dans lequel les moyens de sélection (3) de la source lumineuse (2) permettent la sélection d'au moins une longueur d'onde correspondant au pic du spectre d'absorption du marqueur fluorescent.

8. Dispositif de cartographie du pH intra-rétinien (1) selon la revendication 6 ou la revendication 7, dans lequel les moyens de sélection (3) de la source lumineuse (2) permettent la sélection d'au moins une longueur d'onde correspondant au point isosbestique du spectre d'absorption du marqueur fluorescent.

9. Dispositif de cartographie du pH intra-rétinien (1) selon la revendication 6 ou la revendication 7, dans lequel un filtre est adapté pour filtrer à la longueur d'onde du pic du spectre d'émission de fluorescence.

10. Dispositif de cartographie du pH intra-rétinien selon les revendications 6 à 7 ou la revendication 9, dans lequel un filtre est adapté pour filtrer à la longueur d'onde du point isosbestique du spectre d'émission de fluorescence.

11. Dispositif de photocoagulation des zones de rétine périphériques comprenant :
- un dispositif de cartographie du pH intra-rétinien selon l'une des revendications 6 à 10 ;
- un laser (14) muni de moyens de focalisation et d'orientation (15) d'un faisceau de sortie vers le ou les points de la rétine représentés sur l'image de cartographie du pH fournie par le dispositif (1), par des pixels correspondant à des pH inférieurs à un seuil donné.

## Claims

1. A method of mapping the intraretinal pH of a retina marked with a fluorescent marker having a fluorescence emission spectrum with a pH-dependent amplitude, **characterized by**:
a) illuminating the retina with first excitation radiation having a narrow spectrum centered on a wavelength falling within the excitation spectrum of the fluorescent marker, and forming a first image of the fluorescence emitted by the retina, in the form of a two-dimensional matrix of pixels, at the wavelength of the peak of the fluorescence emission spectrum;
b) illuminating the retina with second excitation radiation having a narrow spectrum centered on a wavelength falling within the excitation spectrum of the fluorescent marker and different from the wavelength of the first radiation, and forming a second image of the fluorescence emitted by the retina, in the form of a two-dimensional matrix of pixels, at the wavelength of the peak of the fluorescence emission spectrum; and
c) calculating, for each pixel, the ratio between the fluorescence levels emitted in response to the two respective excitation wavelengths, said ratio being representative of the pH of the retina, and deriving therefrom a pH map of the retina.

2. The method as claimed in claim 1, wherein the first excitation radiation is centered on the wavelength corresponding to the peak of the absorption spectrum of the fluorescent marker.

3. The method as claimed in claim 1 or claim 2, wherein the second excitation radiation is centered on the wavelength corresponding to the isosbestic point of the excitation spectrum of the fluorescent marker.

4. A method of mapping the intraretinal pH of a retina marked with a fluorescent marker having a fluorescence emission spectrum with a pH-dependent amplitude, **characterized by**:
a) illuminating the retina with excitation radiation having a narrow spectrum centered on a wavelength falling within the excitation spectrum of the fluorescent marker,
b) forming a first image of the fluorescence emitted by the retina, in the form of a two-dimensional matrix of pixels, at the wavelength of the peak of the fluorescence emission spectrum;
c) forming a second image of the fluorescence emitted by the retina, in the form of a two-dimensional matrix of pixels, at a wavelength corresponding to an isosbestic point in the fluorescence emission spectrum; and
d) calculating, for each pixel, the ratio between the fluorescence levels emitted at the two respective wavelengths falling within the excitation spectrum, said ratio being representative of the pH of the retina, and deriving therefrom a pH map of the retina.

5. The method as claimed in claim 4, wherein the excitation radiation is centered on a wavelength corresponding to the peak of the absorption spectrum of the fluorescent marker.

6. A device for mapping the intraretinal pH (1) of a retina marked with a fluorescent marker having a fluorescence emission spectrum with an intensity peak whose height depends on the pH, **characterized in that** it comprises:
- a light source (2) having means (3, 3a, 3b) for selecting only one or two successive different wavelengths of the excitation spectrum of the fluorescent marker;
- a focusing optic (5) for focusing the radiation onto the retina;
- one or more filter(s) adapted for filtering, at one or more given wavelength(s), the fluorescence emission spectrum of the retina illuminated by the light source;
- a 2D-imaging matrix camera (10) providing an image with, at each pixel, a pixel value representative of the fluorescence level;
- means (12) for storing said pixel values;
- means (11) for calculating the ratio between the pixel values at each pixel for two images provided; and
- means (13) for displaying an image, in which each pixel has a value representing the calculated ratio, said ratio being representative of the pH of the point on the retina represented by said pixel.

7. The device for mapping the intraretinal pH (1) as claimed in claim 6, wherein the means (3) for selecting the light source (2) are for selecting at least one wavelength corresponding to the peak of the absorption spectrum of the fluorescent marker.

8. The device for mapping the intraretinal pH (1) as claimed in claim 6 or claim 7, wherein the means (3) for selecting the light source (2) are for selecting at least one wavelength corresponding to the isosbestic point of the absorption spectrum of the fluorescent marker.

9. The device for mapping the intraretinal pH (1) as claimed in claim 6 or claim 7, wherein a filter is adapted for filtering at the wavelength of the peak of the fluorescence emission spectrum.

10. The device for mapping the intraretinal pH (1) as claimed in claims 6 to 7 or claim 9, wherein a filter is adapted for filtering at the wavelength of the isosbestic point of the fluorescence emission spectrum.

11. A device for photocoagulating peripheral areas of a retina, comprising:
- a device for mapping the intraretinal pH as claimed in one of claims 6 to 10;
- a laser (14) having means (15) for focusing and orienting an output beam onto the point or points on the retina that are represented, on the pH mapping image provided by the device (1), by pixels corresponding to pH values below a given threshold value.

## Patentansprüche

1. Verfahren zur Kartographie des intraretinalen pH-Werts einer Netzhaut, die mittels eines fluoreszierenden Markers markiert ist, dessen Fluoreszenzemissionsspektrum eine vom pH-Wert abhängende Amplitude aufweist,
**dadurch gekennzeichnet, dass**
a) die Netzhaut mit einer ersten Anregungsstrahlung mit engem Spektrum bestrahlt wird, welche auf einer dem Anregungsspektrum des fluoreszierenden Markers angehörenden Wellenlänge zentriert ist, und ein erstes Bild der von der Netzhaut emittierten Fluoreszenz gebildet wird, und zwar in der Form einer bidirektionalen Pixelmatrix mit der Wellenlänge der Spitze des Fluoreszenzemissionsspektrums;
b) die Netzhaut mit einer zweiten Anregungsstrahlung mit engem Spektrum bestrahlt wird, welche auf einer dem Anregungsspektrum des fluoreszierenden Markers angehörenden Wellenlänge zentriert ist, welche von derjenigen der ersten Strahlung verschieden ist, und ein zweites Bild der von der Netzhaut emittierten Fluoreszenz gebildet wird, und zwar in der Form der bidimensionalen Pixelmatrix mit der Wellenlänge der Spitze des Fluoreszenzemissionsspektrums;
c) die Beziehung zwischen den Niveaus der jeweils in Antwort auf die beiden Anregungswellenlängen emittierten Fluoreszenz in jedem Pixel berechnet wird, wobei diese Beziehung dem pH-Wert der Netzhaut entspricht, und davon eine Karte des pH-Werts der Netzhaut abgeleitet wird.

2. Verfahren gemäß Anspruch 1, bei welchem die erste Anregungsstrahlung auf der Wellenlänge zentriert ist, die der Spitze des Absorptionsspektrums des fluoreszierenden Markers entspricht.

3. Verfahren gemäß Anspruch 1 oder Anspruch 2, bei welchem die zweite Anregungsstrahlung auf der Wellenlänge zentriert ist, die dem isosbestischen Punkt des Anregungsspektrums des fluoreszierenden Markers entspricht.

4. Verfahren zur Kartographie des intraretinalen pH-Werts einer Netzhaut, die mittels eines fluoreszierenden Markers markiert ist, dessen Fluoreszenzemissionsspektrum eine vom pH-Wert abhängende Amplitude aufweist,
**dadurch gekennzeichnet, dass**
a) die Netzhaut mit einer ersten Anregungsstrahlung mit engem Spektrum bestrahlt wird, welche auf einer dem Anregungsspektrum des fluoreszierenden Markers angehörenden Wellenlänge zentriert ist;
b) ein erstes Bild der von der Netzhaut emittierten Fluoreszenz gebildet wird, und zwar in der Form einer bidirektionalen Pixelmatrix mit der Wellenlänge der Spitze des Fluoreszenzemissionsspektrums;
c) ein zweites Bild der von der Netzhaut emittierten Fluoreszenz gebildet wird, und zwar in der Form einer bidirektionalen Pixelmatrix mit einer Wellenlänge, die einem isosbestischen Punkt des Fluoreszenzemissionsspektrums entspricht;
d) die Beziehung zwischen den Niveaus der Fluoreszenz in jedem Pixel berechnet wird, die jeweils mit den beiden, dem Emissionsspektrum angehörenden Anregungswellenlängen emittiert wird, wobei diese Beziehung dem pH-Wert der Netzhaut entspricht, und davon eine Karte des pH-Werts der Netzhaut abgeleitet wird.

5. Verfahren gemäß Anspruch 4, bei welchem die erste Anregungsstrahlung auf der Wellenlänge zentriert ist, die der Absorptionsspektrumsspitze des fluoreszierenden Markers entspricht.

6. Vorrichtung (1) zur Kartographie des intraretinalen pH-Werts einer Netzhaut, die mittels eines fluoreszierenden Markers markiert ist, dessen Fluoreszenzemissionsspektrum eine Intensitätsspitze aufweist, deren Höhe vom pH-Wert abhängt,
**dadurch gekennzeichnet, dass** sie einschließt:
- eine Lichtquelle (2), die mit Mitteln (3, 3a, 3b) versehen ist, welche die Wahl von einer einzigen oder von zwei aufeinanderfolgenden, verschiedenen Wellenlängen des Anregungsspektrums des fluoreszierenden Markers zulässt,
- eine Fokussierungsoptik (5), die das Fokussieren der Strahlung auf die menschliche Netzhaut zulässt,
- einen oder mehrere Filter (6), der/die dazu angepasst ist/sind, das Fluoreszenzemissionsspektrum der mittels der Lichtquelle bestrahlten Netzhaut bei einer oder mehreren gegebenen Wellenlängen zu filtern,
- eine Matrixkamera (10) zum Bilden eines 2D-Bilds, die ein Bild liefert, das in jedem Pixel einen das Fluoreszenzniveau repräsentierenden Wert aufweist,
- Mittel (12) zum Speichern dieser Werte,
- Mittel (11) zum Berechnen der Beziehung zwischen den Werten in jedem Pixel für die beiden gelieferten Bilder,
- Mittel (13) zum Visualisieren eines Bilds, von welchem jedes Pixel einen Wert aufweist, der die berechnete Beziehung darstellt, wobei diese Beziehung für den pH-Wert des mittels des Pixels dargestellten Punkts der Netzhaut repräsentativ ist.

7. Vorrichtung (1) zur Kartographie des intraretinalen pH-Werts gemäß Anspruch 6, bei welcher die Mittel (3) zum Wählen der Lichtquelle (2) die Wahl wenigstens einer Wellenlänge zulässt, die der Absorptionsspektrumsspitze des fluoreszierenden Markers entspricht.

8. Vorrichtung (1) zur Kartographie des intraretinalen pH-Werts gemäß Anspruch 6 oder Anspruch 7, bei welcher die Mittel (3) zum Wählen der Lichtquelle (2) die Wahl wenigstens einer Wellenlänge zulässt, die dem isosbestischen Punkt des Absorptionsspektrums des fluoreszierenden Markers entspricht.

9. Vorrichtung (1) zur Kartographie des intraretinalen pH-Werts gemäß Anspruch 6 oder Anspruch 7, bei welcher der Filter dazu angepasst ist, bei der Wellenlänge der Spitze des Fluoreszenzemissionsspektrums zu filtern.

10. Vorrichtung zur Kartographie des intraretinalen pH-Werts gemäß Ansprüchen 6 bis 7 oder Anspruch 9, bei welcher der Filter dazu angepasst ist, bei der Wellenlänge des isosbestischen Punkts des Fluoreszenzemissionsspektrums zu filtern.

11. Vorrichtung zur Photokoagulation der Umfangsbereiche der Netzhaut, umfassend:
- eine Vorrichtung zur Kartographie des intraretinalen pH-Werts gemäß einem der Ansprüche 6 bis 10:
- einen Laser (14), der mit Mitteln (15) zum Fokussieren und Ausrichten eines Austrittsbündels in Richtung des Punkts oder der Punkte der Netzhaut, der/die auf dem Kartographiebild des pH-Werts dargestellt ist/sind, welches mittels der Vorrichtung (1) anhand von Pixeln gefertigt worden ist, die pH-Werten entsprechen, die niedriger als eine gegebene Schwelle sind.
